# FASCICULE DE BREVET EUROPEEN

(11) **EP 2 895 628 B1**
(45) Date de publication et mention de la délivrance du brevet: **10.10.2018**
(21) Numéro de dépôt: 13776529.3
(22) Date de dépôt: 12.09.2013
(51) Int. Cl.: C12P 7/64, A23D 9/00, C12R 1/645

(54) **HUILE ENRICHIE EN ACIDE ARACHIDONIQUE ISSUE DE MICROORGANISMES (CHAMPIGNON UNICELLULAIRE MORTIERELLA ALPINA) ET SON PROCEDE DE PREPARATION**
MIT ARACHIDONSÄURE ANGEREICHERTES ÖL AUS MIKROORGANISMEN (EINZELLIGER PILZ MORTIERELLA ALPINA) UND VERFAHREN ZU SEINER HERSTELLUNG
OIL ENRICHED WITH ARACHIDONIC ACID OF MICROORGANISMS (UNICELLULAR FUNGUS MORTIERELLA ALPINA) AND METHOD FOR THE PRODUCTION THEREOF

(30) Priorité: 14.09.2012 CN 201210343057
(43) Date de publication de la demande: 22.07.2015
(73) Titulaire: Roquette Frères, 62136 Lestrem (FR)
(72) Inventeur: QIAN, Yun, Wuhan 430056 (CN); ZHOU, Jie, Wuhan 430056 (CN); PORA, Bernard, Wuhan (Economic Develpt Area) 430056 (CN)
(74) Mandataire: Cabinet Plasseraud
(86) Numéro de dépôt international: PCT/FR2013/052092
(87) Numéro de publication internationale: WO 2014/041303

(56) Documents cités:
- EP-A1- 0 956 774
- EP-A1- 1 035 211
- WO-A1-2004/084882
- DATABASE CBS 29 octobre 2013 (2013-10-29), "Mortierella alpina Peyronel, I germi astmosferici dei fungi con micelio: 17 (1913) [MB#170280]", XP002716227, Database accession no. 529.72 -& DATABASE EMBL [Online] 18 juin 2013 (2013-06-18), "Mortierella alpina strain CBS 529.72 28S ribosomal RNA gene, partial sequence.", XP002716228, extrait de EBI accession no. EM_STD:KC018320 Database accession no. KC018320
- EIJI SAKURADANI ET AL: "Improvement of arachidonic acid production by mutants with lower n-3 desaturation activity derived from Mortierella alpina 1S-4", APPLIED MICROBIOLOGY AND BIOTECHNOLOGY, vol. 66, no. 3, 5 août 2004 (2004-08-05), pages 243-248, XP055087551, ISSN: 0175-7598, DOI: 10.1007/s00253-004-1682-7
- YOSHIFUMI SHINMEN ET AL: "Production of arachidonic acid byfungi ; Selection of a potent producer and optimization of culture conditions for large-scale production", APPLIED MICROBIOLOGY AND BIOTECHNOLOGY, vol. 31, no. 1, juillet 1989 (1989-07), pages 11-16, XP035169384, SPRINGER, BERLIN, DE ISSN: 1432-0614, DOI: 10.1007/BF00252518
- CERTIK M ET AL: "Desaturase-defective fungal mutants: useful tools for the regulation and overproduction of polyunsaturated fatty acids", TRENDS IN BIOTECHNOLOGY, vol. 16, no. 12, décembre 1998 (1998-12), pages 500-505, XP004143810, ELSEVIER PUBLICATIONS, CAMBRIDGE, GB ISSN: 0167-7799, DOI: 10.1016/S0167-7799(98)01244-X

## Description

La présente invention se rapporte à une nouvelle composition d'huile riche en acide arachidonique, huile produite par un microorganisme, préférentiellement produite par un champignon unicellulaire (moisissure filamenteuse) du genre *Mortierella,* en l'occurrence une souche de *Mortierella alpina* particulière.

Les lipides constituent une des trois grandes familles de macronutriments avec les protéines et les glucides.

Parmi les lipides, on distingue notamment les triglycérides et les phospholipides.

Les triglycérides représentent environ 95 % des lipides alimentaires ingérés. Dans l'organisme, ils sont présents principalement dans les tissus adipeux et constituent la forme principale de stockage de l'énergie.

Les phospholipides sont des lipides de structure car ils sont des constituants des membranes cellulaires dont ils assurent entre autre la fluidité.

Les triglycérides et phospholipides sont composés majoritairement d'acides gras qui sont à la fois apportés par l'alimentation et, pour certains d'entre eux, synthétisés par l'organisme.

La classification biochimique (basée sur le nombre de doubles liaisons contenues dans la molécule d'acide gras) distingue les acides gras saturés (AGS), les acides gras monoinsaturés (AGMI) et les acides gras polyinsaturés (AGPI).

Du point de vue physiologique, on distingue :
- les acides gras indispensables, nécessaires au développement et au bon fonctionnement du corps humain, mais que notre corps ne sait pas fabriquer ;
- les acides gras dit « conditionnellement » indispensables, essentiels pour la croissance normale et les fonctions physiologiques des cellules mais qui peuvent être fabriqués à partir de leur précurseur s'il est apporté par l'alimentation. Ils sont donc rigoureusement requis si leur précurseur indispensable est absent.
- Les acides gras non indispensables.

L'ensemble des acides gras indispensables et « conditionnellement » indispensables constituent les acides gras essentiels.

Les autres acides gras sont dits non-essentiels.

On distingue deux grandes familles d'acides gras essentiels : les acides gras oméga 6 (ou AGPI n-6), dont le précurseur et le représentant majeur est l'acide linoléique (LA) et les acides gras oméga 3 (ou AGPI n-3) dont le précurseur est l'acide alpha-linolénique (ALA).

Parmi les acides gras non indispensables, on trouve notamment :
- l'acide eicosapentaénoïque (EPA) de la famille des acides gras oméga 3,
- l'acide oléique, acide gras monoinsaturé majoritaire dans notre alimentation, et
- les acides gras saturés.

Outre leur production à partir d'arachides, de carthame, de colza, de maïs, de lin, de noix, de sésame, de soja, de tournesol, une grande variété d'acides gras polyinsaturés peut être produite par différents organismes unicellulaires (algues, champignons, etc.).

L'acide arachidonique (« ARA ») est un acide gras à chaîne longue que l'on trouve dans certaines huiles végétales.

C'est un acide gras polyinsaturé en C 20:4 (n-6, n-9, n-12, n-15) i.e. un acide gras à 20 atomes de carbone et quatre liaisons éthyléniques situées sur les atomes de carbone n°5 (n-15), n°8 (n-12), n°11 (n-9) et n°14 (n-6).

C'est un acide gras également dit « tétraènoïque » car il possède 4 insaturations (4 doubles liaisons Carbone-Carbone).

On le trouve également dans la littérature sous la dénomination « toutes cis 5,8,11,14-eicosatétraènoïque » (« cis » définissant la configuration des doubles liaisons, « toutes » les doubles liaisons étant en configuration « cis »).

L'ARA est l'un des AGPI en C20 le plus abondant dans le corps humain, où il est fabriqué à partir de l'acide linoléique.

Il est retrouvé dans les organes, les muscles et le sang.

L'ARA est un précurseur important d'une grande variété de composés biologiquement actifs, connus collectivement sous le terme « eicosanoïdes », un groupe comprenant les prostaglandines, thromboxanes et leucotriènes.

Ces eicosanoïdes présentent des effets régulateurs sur le métabolisme des lipoprotéines, de la rhéologie du sang, du tonus musculaire, de la fonction des leucocytes, de l'activation plaquettaire et de la croissance cellulaire.

L'ARA est également l'un des composants de la fraction lipidique du lait maternel humain et est considéré comme essentiel pour le développement neurologique optimal chez les nourrissons.

Dans leur effort pour obtenir des préparations pour nourrissons correspondant au profil des acides gras à longue chaîne du lait maternel, les scientifiques et organismes de réglementation des aliments ont recommandé que l'acide arachidonique soit ajouté aux préparations pour nourrissons, en particulier dans la formule utilisée pour les bébés prématurés.

En particulier, il est préférable que l'huile contenant de l'acide arachidonique produite pour son utilisation dans des préparations pour nourrissons contiennent peu ou pas d'autres AGPI (par exemple, l'acide eicosapentaénoïque ou EPA).

Ces autres AGPI ne sont en effet pas recommandés parce que certains de ces acides gras peuvent interférer avec l'utilisation de l'acide arachidonique en tant que tel par l'enfant, et/ou peut gêner le mélange de l'huile contenant l'acide arachidonique avec d'autres huiles pour atteindre le ratio approprié des acides gras du lait maternel (lait maternel reconstitué) ou pour d'autres applications souhaitées.

Par ailleurs, l'ARA a une grande variété d'applications telles que l'utilisation dans les préparations pour les denrées alimentaires chez l'Homme mais aussi comme aliments pour animaux.

L'ARA a en effet plusieurs propriétés reconnues en nutrition animale :
- propriétés anti-inflammatoires, car l'ARA est un précurseur des prostaglandines série II et de l'anandamide (principalement démontrées sur porcs),
- propriétés anti-stress chez le poisson (dorade) par un autre biais que les prostaglandines,
- propriétés immuno-modulatrices chez les porcs.

Des études de transfert ont également été réalisées pour visualiser le passage de l'acide arachidonique dans la chair, le lait ou les oeufs produits.

Lorsqu'il est isolé à partir de microorganismes, le produit commercial est notamment disponible sous la forme d'une huile riche en acide arachidonique obtenue grâce à la fermentation de moisissures filamenteuses du genre *Mortierella.*

Dans EP 276541, il est ainsi décrit un procédé de production d'ARA par *Mortierella elongata, Mortierella exigua* et *Mortierella hygrophila.*

La demande de brevet internationale WO 94/28913 décrit quant à elle un procédé fermentaire utilisant *Mortierella alpina* pour produire de l'ARA substantiellement dépourvu d'EPA. Sakuradani et al (2004, Applied Microbiology and Biotechnology, 66, 243-248), Shinmen et al (1989, Applied Microbiology and Biotechnology, 31, 11-16) and Certik et al (1998, Trends in Biotechnology, 16, 500-505) décrivent des procédés utilisant *Mortierella alpina* et produisant au minimum 50% d'ARA. EP 1 035 211 décrit une souche de *Mortierella alpina* obtenue par mutagenèse et présentant de bonnes capacités de production d'ARA.

Cependant, comme il est affirmé dans EP 726.321, de toutes les *Mortierella* testées pour leur production en ARA (*alpina, hygrophila, spinosa, schmuckeri et carmagensis*)*,* ce sont les souches de *Mortierella schmuckeri* ou *Mortierella carmagensis* qui présentent la productivité la plus avantageuse.

Il est donc admis des spécialistes du domaine de la production d'ARA que c'est par le choix d'une souche de *M. Schmuckeri* ou *M. carmagensis* que l'on peut garantir les meilleurs rendements, productivité et qualité, au détriment des *M. alpina, hygrophila ou spinosa.*

**Cependant, il demeure un besoin de disposer de moyens alternatifs permettant de produire des huiles de qualité, à haute tenue en ARA, et présentant des profils tout à fait particuliers en acides gras saturés ou polyinsaturés à longue chaîne.**

Par ailleurs, pour de nombreuses Autorités Réglementaires nationales, seule *Mortierella alpina* est autorisée en alimentation infantile.

Il a été tout d'abord du mérite de la société Demanderesse de proposer une nouvelle composition d'huile, enrichie en acide arachidonique, présentant :
- de faibles teneurs en acide gras polyinsaturés autres que l'ARA (tel que l'EPA),
- une teneur limitée en certains acides gras saturés à longue chaîne (tels que l'acide béhénique, l'acide myristique, l'acide palmitique, ou l'acide lignocérique).

Il a par ailleurs été remarqué par la société Demanderesse que la faible teneur en ces acides gras saturés permet d'obtenir une huile de meilleure qualité que celles du commerce, en termes de limpidité et de faible turbidité à froid.

Soucieuse également de mettre au point un procédé de production bien plus efficace et bien moins couteux que ceux décrits dans l'état de la technique, la société Demanderesse a, au cours de ses recherches, identifié une nouvelle souche de *Mortierella alpina* présentant une capacité exceptionnelle de production d'acide arachidonique puisqu'elle permet une production atteignant plus de 50 % d'acide arachidonique (les % s'entendant ici en poids d'acides gras totaux).

La société Demanderesse a donc été à l'encontre d'un préjugé technique, qui vise à rechercher dans les souches de type *M. Schmuckeri* ou *M. carmagensis* les meilleures souches productrices d'ARA.

Par ailleurs, outre la capacité remarquable de production d'acide arachidonique, cette souche permet également d'obtenir (les % s'entendant ici en poids d'acides gras totaux). :
- moins de 0,5 %, de préférence moins de 0,2 % d'EPA,
- moins de 0,5 %, de préférence moins de 0,2 % d'acide myristique (C14:O),
- moins de 9 %, de préférence moins de 7 % d'acide palmitique (C16:0),
- moins de 3 %, de préférence moins de 2,5 % d'acide béhénique (22:0), et
- moins de 3 %, de préférence moins de 2,5 % d'acide lignocérique (C 24:0).

Cette souche de *Mortierella alpina* a été déposée en France le 12 juin 2012 auprès de la Collection Nationale de Cultures de Microorganismes de l'Institut Pasteur (CNCM) sous le numéro CNCM I-4642, mais également en Chine auprès du CHINA CENTER FOR TYPE CULTURE COLLECTION (CCTCC) de l'université de Wuhan, Wuhan 430072, P.R. China sous le numéro M 209116.

Elle a été caractérisée par séquençage de la région D1 - D2 du gène codant pour l'ARN 25S (SEQ ID No 1):

Ceci a permis de l'identifier comme étant une souche du type *Mortierella alpina.*

Par conséquent, la présente invention est relative à la souche de *Mortierella alpina* déposée le 12 juin 2012 auprès de la CNCM sous le numéro I-4642.

Cette souche pourra être désignée « CNCM I-4642 » ultérieurement dans la présente demande.

La présente description décrit également un variant de cette souche ou une souche dérivée de celle-ci, ledit variant ou ladite souche dérivée conservant la propriété de produire des teneurs en acide arachidonique élevées. En particulier, elle permet une production d'ARA d'au minimum 50 % d'acide arachidonique en poids d'acides gras totaux.

En particulier, elle concerne une souche de *Mortierella alpina* obtenue à partir de la souche CNCM I-4642 par mutagenèse ou par transformation génique. La mutagenèse peut être dirigée et/ou aléatoire.

La présente description décrit aussi une méthode de préparation d'une telle souche comprenant la mutagenèse ou la transformation génique de la souche CNCM I-4642 et facultativement une étape de criblage permettant de sélectionner les souches produisant au moins 50 % d'acide arachidonique en poids d'acides gras totaux.

La présente description décrit une méthode de culture de la souche CNCM I-4642 ou d'un variant ou souche dérivée de celle-ci conservant la capacité de production d'acide arachidonique, comprenant une étape de culture de cette souche dans un milieu approprié et dans des conditions de fermentation adaptées.

La présente description décrit par ailleurs une méthode de préparation de l'acide arachidonique sous la forme d'une huile extraite de la souche CNCM I-4642 ou d'un variant ou souche dérivée de celle-ci, caractérisée en ce que l'huile contenant l'acide arachidonique est préparée par une méthode comprenant :
∘ culture de la souche en conditions hétérotrophiques de manière à produire une biomasse présentant entre 40 et 55 % en poids de lipides, préférentiellement entre 40 et 50 % en poids de lipides, plus préférentiellement encore de l'ordre de 45 % en poids de lipides et entre 50 et 55 % en poids d'ARA par rapport au poids d'acides gras totaux.
∘ récolte de la biomasse ainsi préparée,
∘ séchage de ladite biomasse,
∘ extraction de l'huile par solvant choisi dans le groupe constitué de l'hexane et du butane, plus particulièrement par du butane liquide, et,
∘ raffinage et récupération de l'huile ainsi extraite.

De manière optionnelle, la biomasse résultant de l'étape d'extraction de l'huile peut-être elle-même récupérée et valorisée en nutrition animale (comme complément alimentaire pour animaux de ferme (cochons...), animaux de compagnie et en aquaculture.)

La culture est réalisée en conditions hétérotrophiques. Généralement, l'étape de culture comprend une étape de préculture, pour revivifier la souche, puis une étape de culture ou de fermentation proprement dite. Cette dernière étape correspond à l'étape de production des composés lipidiques d'intérêt.

La société Demanderesse recommande, pour la souche CNCM I-4642, de mettre en oeuvre une fermentation aérobie en cinq étapes, comme il sera exemplifié ci-après.

Les quatre premières étapes sont caractérisées par une culture de la souche CNCM I-4642 dans un milieu dans lequel l'apport en source de carbone est régulé en fonction de la cinétique de production de la biomasse, des lipides totaux et de l'acide arachidonique.

Dans ces quatre étapes, il est à noter que, contrairement à d'autres procédés de l'état de l'art pour la production d'acide arachidonique par fermentation de *Mortierella,* l'alimentation en source de carbone n'est pas ou peu limitante en ce qui concerne la croissance du micro-organisme.

Par contre, au cours de la cinquième étape, le milieu de culture présente une faible concentration en source de carbone, au maximum 1 % en poids et l'alimentation en source de carbone est même arrêtée.

Dans cette étape, la source de carbone devient limitante pour la croissance du micro-organisme, ou est limitée de telle sorte que les micro-organismes sont conduits à métaboliser leurs propres matières grasses et/ou lipides.

Il est important de noter que contrairement à ce qui est décrit dans la littérature :
- il n'est pas utile ici de réguler/contrôler la concentration en oxygène dissous dans le milieu de culture, mais plutôt de la maintenir au maximum. Il n'y a donc pas ici de régulation dans l'apport en oxygène pour augmenter la production de l'ARA.
- il n'est pas non plus utile de définir précisément les apports en phosphates, en potassium, en sodium, en magnésium et en calcium dans le milieu de culture, pour notamment contrôler la morphologie du mycélium du micro-organisme pendant la fermentation.

La présente description décrit ensuite la récupération, en fin de fermentation, de la biomasse riche en composés lipidiques d'intérêt, en l'occurrence ici l'ARA.

Après l'étape de fermentation, la biomasse peut être :
- pasteurisée, de manière à inactiver les enzymes de dégradation des lipides (lipases) présentes dans la biomasse en tant que telle, mais aussi dans le milieu de culture,
- récupérée du milieu de fermentation par toute méthode connue en soi de l'homme du métier, par exemple la biomasse peut être extraite du fermenteur et simplement concentrée par microfiltration ou centrifugation, ou lavée par succession de concentrations-dilutions avec une solution aqueuse.

La présente description est relative à la biomasse comprenant la souche CNCM I-4642 ou d'un variant ou d'une souche dérivée de celle-ci conservant la capacité de production d'ARA.

Tout particulièrement, après l'étape de fermentation ou culture, cette biomasse est riche en composés lipidiques d'intérêt tels que l'ARA.

Elle est susceptible d'être obtenue par la méthode décrite dans le présent document.

Après fermentation, la biomasse peut contenir :
- entre 40 et 55 % en poids de lipides par rapport au poids total de la biomasse, préférentiellement entre 40 et 50 % en poids de lipides, plus préférentiellement encore de l'ordre de 45 % en poids de lipides et
- entre 50 et 55 % en poids d'ARA par rapport au poids d'acides gras totaux.

Outre la biomasse, la présente description est également relative à un extrait ou lysat cellulaire, préparé à partir de cette biomasse comprenant la souche CNCM I-4642 ou d'un variant ou d'une souche dérivée de celle-ci conservant la capacité de production d'ARA.

En particulier, cet extrait ou lysat est préparé à partir de la biomasse récupérée après fermentation.

Cet extrait ou ce lysat est alors riche en composés lipidiques d'intérêt tels que l'ARA. Notamment, il pourra contenir entre 40 et 55 % en poids de lipides, préférentiellement entre 40 et 50 % en poids de lipides, plus préférentiellement encore de l'ordre de 45 % en poids de lipides et entre 50 et 55 % en poids d'ARA par rapport au poids d'acides gras totaux, et entre 50 et 55 % en poids d'ARA par rapport au poids d'acides gras totaux.

La rupture des cellules pour l'extraction du contenu lipidique peut être effectuée par différentes voies parmi lesquelles les voies mécanique, chimique, et enzymatique.

La présente description est également relative à l'huile extraite de la biomasse par un solvant choisi dans le groupe constitué de l'hexane et du butane, plus particulièrement par du butane liquide, notamment en plusieurs extractions successives.

L'huile peut être récupérée après distillation sous vide.

Cependant, de préférence, l'huile est ensuite récupérée après deux phases de raffinage et de purification.

La phase de raffinage comprend six étapes successives classiquement mises en oeuvre par l'homme du métier:
- dégommage : acidification à l'acide citrique,
- saponification : neutralisation avec alcalis,
- centrifugation pour éliminer les gommes et les savons,
- lavage à l'eau,
- décoloration avec de la terre de silice, du charbon actif et de l'argile, et
- filtration.

La phase de purification a pour objectif d'éliminer les mauvais goûts, les peroxydes et optimise la stabilité de l'huile.

Cette phase comprend successivement une ou deux étapes de :
- distillation moléculaire (utilisée si la teneur en insaponifiables (facteur UNS) est trop élevée),
- désodorisation à la vapeur sous vide poussé.

Comme il sera exemplifié ci-après, la société Demanderesse recommande de récupérer également la fraction volatile issue de l'étape de désodorisation à la vapeur sous vide poussé.

En effet, cette fraction est constituée d'au moins 80 % de triglycérides (dont la distribution en acides gras est comparable à celle de l'huile d'ARA raffinée) et contient de l'ordre de 10 % en squalène.

Ainsi, la méthode de production de l'ARA comprend :
- la récolte de la biomasse,
- le séchage de la biomasse ou la préparation du lysat cellulaire,
- l'extraction de l'huile, et
- le raffinage et la purification de l'huile.

L'huile est extraite de la biomasse microbienne sèche provenant d'un bouillon de fermentation pasteurisé.

La biomasse est récoltée et séchée, puis on extrait l'huile de la biomasse sèche en utilisant par exemple le butane liquide comme solvant.

La biomasse résiduelle est récupérée, séchée et conditionnée pour des applications en nutrition animale.

Finalement, l'huile présente au moins 50% d'ARA et au moins 90% de triglycérides par rapport au poids d'acides gras totaux.

La présente description est enfin relative à l'utilisation de l'ARA ou de l'huile riche en ARA, produit par l'un quelconque des procédés de la présente description, dans la préparation de compositions destinées aux domaines alimentaires, notamment l'alimentation infantile.

Ainsi, elle concerne une méthode de préparation de compositions destinées aux domaines alimentaires comprenant la production d'ARA ou de l'huile riche en ARA par l'un quelconque des procédés de la présente description, puis la préparation de compositions destinées aux domaines alimentaires en ajoutant ladite ARA ou huile riche en ARA.

La présente description est en particulier relative à un produit ou une composition comprenant la souche CNCM I 46-42 ou d'un variant ou d'une souche dérivée de celle-ci conservant la capacité de production de l'ARA, une biomasse obtenue après culture ou fermentation de celle-ci, un extrait ou lysat cellulaire de celle-ci. Elle concerne également un produit ou une composition comprenant de l'huile riche en ARA produite par l'un quelconque des procédés de la présente description. Ledit produit ou ladite composition est riche en en composés lipidiques d'intérêt, tel l'acide arachidonique (ou ARA). En particulier, Ledit produit ou ladite composition est riche en ARA. De préférence, il ou elle comprend plus de 50 % d'acide arachidonique (ou ARA) en poids sur acide gras totaux. En outre, il ou elle présente :
- moins de 0,5 %, de préférence moins de 0,2 % d'EPA,
- moins de 0,5 %, de préférence moins de 0,2 % d'acide myristique (C14:O),
- moins de 9 %, de préférence moins de 7 % d'acide palmitique (C16:0),
- moins de 3 %, de préférence moins de 2,5 % d'acide béhénique (C22:0) et
- moins de 3 %, de préférence moins de 2,5 % d'acide lignocérique (C 24:0).

De préférence, ce produit ou cette composition est une composition alimentaire ou un complément alimentaire ou nutritionnel.

Il peut être sous forme liquide ou solide.

En particulier, le produit peut contenir un lyophilisat de cellules ou extrait ou lysat cellulaire de celle-ci.

Ce produit ou cette composition peut être sous forme de poudre, granule, gélule, capsule, ou cachet, de préférence sous forme de poudre.

Alternativement, le produit ou la composition est sous forme liquide et comprend l'huile brute ou raffinée obtenue par l'un quelconque des procédés de la présente description.

Quant à la biomasse résiduelle résultant de l'étape d'extraction de l'huile, elle présente une composition tout à fait adaptée à sa destination en nutrition animale, comme il sera exemplifié ci-après.

L'invention sera mieux comprise à l'aide des exemples qui suivent, lesquels se veulent illustratifs et non limitatifs.

### Exemple 1 : Production d'une huile brute riche en ARA par la souche de Mortierella alpina CNCM I-4642

La fermentation principale est effectuée dans des fermenteurs contenant des milieux de culture ensemencés à partir de deux séries de fermenteurs de plus petite taille :

### Préculture rapide (6 x 200 ml)

- dans un milieu contenant 3 % de glucose, 1,5 % de poudre de levure (contenu en azote de 7 %),
- température de 28°C,
- agitation de 240 rpm,
- Durée de 24 - 48 heures,

Au terme de cette préculture : les 6 x 200 ml sont mélangés ensemble dans un réacteur de 3 l.

### Première étape de fermentation (réacteur de 1 m³)

- dans un milieu contenant 3 % de glucose, 1,5 % de poudre de levure (contenu en azote de 7 %),
- taux d'inoculum : 0,1 à 1 % vol,
- Tp : 28°C,
- Pression : 0,03 mPa,
- Taux d'aération : maximum,
- Pas d'agitation mécanique,
- Durée : 32 à 48 h,
- Volume utile : 70 % du volume total,
- pH : 6 à 6,5 (ajusté avec alcalis),
- Volume final : 750 l.

### Deuxième étape de fermentation (réacteur de 10 m³)

- dans un milieu contenant 4 % de glucose, 1,5 % de poudre de levure (contenu en azote de 7 %), antimousse 0,2 % (huile de tournesol),
- taux d'inoculum : 10 à 15 % vol,
- Tp : 28°C,
- Pression : 0,03 mPa,
- Taux d'aération : maximum,
- Pas d'agitation mécanique,
- Durée : 24 à 32 h,
- Volume utile : 70 % du volume total,
- pH : 6 à 6,5 (ajusté avec alcalis),
- Volume final : 7,5 m³.

### Fermentation principale (réacteur de 85 m³)

- dans un milieu contenant 3 % de glucose, 2 % de poudre de levure (contenu en azote de 7 %), 0,2 % (huile de tournesol),
- Rapport C/N (Carbone / Azote): 7 à 8,
- Taux d'inoculum : 15 à 25 % vol,
- Tp : 27 - 28 °C,
- Taux d'aération : maximum,
- Pas d'agitation mécanique,
- Durée : 155 à 165 h,
- Volume utile : 70 % du volume total,
- pH : 7 - 7,1,
- volume final : 60 - 63 m³.

Si l'on s'attache à décrire cette étape de fermentation principale, on retient qu'il s'agit ici d'un processus de fermentation aérobie.

Le réacteur de fermentation est défini de manière à effectuer l'aération et le mélange des cellules et du milieu de culture sans moyen mécanique : il est préféré ici une colonne à bulles (« bubble column ») d'un diamètre de 3,5 m et d'une hauteur de 8,7 m.

Le volume total du fermenteur est de 85 m³. La fermentation dure généralement 6 à 7 jours (155 à 165 heures) et la température est de 27 à 28 °C.

Un milieu approprié mais simple est utilisé dans la fermentation.

La source de carbone préférée est uniquement constituée de glucose et la source d'azote est constituée de poudre de levure (7% de teneur en N).

Le rapport molaire C / N est maintenue de 7 à 8.

Les sources d'azote et de carbone sont stérilisées séparément et ajoutée séparément.

La source d'azote est fournie en une seule fois au début de la fermentation du glucose et est ajoutée en mode "fed batch".

Le milieu de culture est de l'eau qui contient :
- l'agent antimousse (à base de silicone de qualité alimentaire) et / ou de l'huile de tournesol pour contrôler le niveau de mousse produite et
- l'hydroxyde de sodium (NaOH) pour contrôler le pH à une valeur optimale comprise entre 6,5 à 7,1.

L'optimum de température est de préférence de 27 à 28°C.

Le milieu est agité pendant la fermentation.

Ceci est réalisé par une aération produite par "barbotage" d'air stérilisé dans le milieu et qui fournit de l'oxygène aux cellules.

L'aération n'est pas contrôlée et est maintenue maximale pendant toute la durée de la fermentation à une valeur comprise entre 0,8 à 1 VVM.

Il n'y a pas d'agitation mécanique supplémentaire pour favoriser l'aération.

A la fin de la fermentation, lorsque la teneur en glucose est égale à zéro et l'augmentation du pH à plus de 7,5, (ce qui indique un début de lyse des cellules), le réacteur est arrêté et les micro-organismes peuvent alors être retirés de la cuve de fermentation grâce à une filtration (filtre-presse).

Pendant la fermentation dans le réacteur de 85 m³, toute la quantité de source de carbone présent dans le milieu est contrôlée et cinq étapes ont été clairement identifiées comme décrit ci-après :

### 1. Première étape

Celle-ci commence à partir de t = 0 h et se termine généralement après 20 à 24 heures.

Pendant cette période, la source de carbone est en excès et ne doit pas être limitante pour la croissance des cellules.

La source de carbone n'est pas ajoutée pendant cette période et le contenu en sucres réducteurs du milieu diminue de 30 à 20 g/l.

Le pH n'est pas contrôlé ; sa valeur diminue légèrement de 6,3 à 5,7.

À la fin de cette première étape, la concentration en biomasse peut atteindre 16 g/l de milieu, le contenu en lipides totaux est d'au moins 4,7 g/l de milieu, et la teneur en acide arachidonique est de préférence plus de 1,3 g/l de milieu.

### 2. Deuxième étape

Celle-ci commence à t = 20 heures et se termine généralement après 50 à 55 heures.

Pendant cette période, la source de carbone disponible ne doit pas être limitant et la vitesse d'ajout doit normalement être légèrement inférieure au taux de consommation des cellules.

Une solution de glucose (25 g/l) est ajoutée à un taux moyen de 0,15 M de carbone/kg de milieu et par heure (l'unité est une quantité molaire de carbone dans la source de carbone) et le contenu en sucres réducteurs dans le milieu diminue de 20 à 11 g/l.

Le pH est ajusté et contrôlé de 5,7 à 7 en alimentant par une solution de NaOH.

À la fin de cette deuxième étape, la concentration de la biomasse peut atteindre 21 g/l de milieu, la teneur totale en lipides est d'au moins 8,3 g/l de milieu et la teneur en acide arachidonique est de préférence de plus de 3,1 g/l de milieu.

Ces deux premières étapes ont pour objectif de produire la quantité principale de biomasse.

### 3. Troisième étape

Celle-ci commence à t = 55 heures et se termine généralement au bout de 100 à 105 heures.

Pendant cette période, la source de carbone disponible n'est toujours pas limitant et le taux d'ajout doit généralement être inférieur au taux de consommation par les cellules.

Une solution de glucose (25 g/l) est ajoutée à un taux moyen de 0,08 M / kg de milieu et par heure) et le contenu en sucres réducteurs diminue de 11 à 4 g/l.

Le pH est ajusté et contrôlé à une valeur de 7 à 7,1 en ajoutant une solution de NaOH

À la fin de cette troisième étape, la concentration de la biomasse peut atteindre 24 g/l, la teneur totale en lipides est d'au moins 11 g/l et la teneur en acide arachidonique de préférence de plus de 5 g/l de milieu.

### 4. Quatrième étape

Celle-ci commence à t = 100 heures et se termine généralement après 130 à 135 heures.

Pendant cette période, la source de carbone est légèrement limitée et le taux d'addition doit généralement être inférieur au taux de consommation par les cellules.

Une solution de glucose (25 g/l) est ajoutée à un taux moyen de 0,04 M de carbone / kg de milieu par heure et le contenu en sucres réducteurs diminue de 4 à 1 g/l.

Le pH n'est pas contrôlé et augmente légèrement, passant de 7,1 à 7,3.

À la fin de cette quatrième étape, la concentration de la biomasse peut atteindre 26 g/l de milieu, la teneur totale en lipides est d'au moins 12,4 g/l de milieu et la teneur en acide arachidonique est de préférence de plus de 6,2 g/l de milieu.

La troisième et la quatrième étapes ont pour objectif de produire la quantité principale de lipides.

### 5. Cinquième étape

Celle-ci commence à t = 130 heures et se termine généralement au bout de 160 à 170 heures.

Pendant cette période, la source de carbone disponible est limitant et l'alimentation en source de carbone est arrêtée.

La teneur en sucres réducteurs dans le milieu diminue rapidement de 1 à 0 g/l.

Le pH n'est pas contrôlé et augmente légèrement de 7,3 à 7,5.

A la fin de cette cinquième étape, la concentration de la biomasse peut atteindre 27 g/l de milieu, la teneur en lipides totaux est d'au moins 14 g/l, et la teneur en acide arachidonique est de préférence de plus de 7,6 g/l de milieu.

Cette cinquième étape a pour objectif d'augmenter le contenu en acide arachidonique sans affecter la concentration totale en lipides.

Quand le pH dépasse la valeur de 7,5, qui indique le début de la lyse cellulaire, le réacteur est arrêté, le milieu de fermentation est pasteurisé pendant 30 min à 70°C, et après refroidissement à 30°C - 25°C, les microorganismes sont retirées du fermenteur par passage à travers un filtre presse.

Les principaux indicateurs de cette conduite de fermentation sont présentés dans le tableau I suivant :

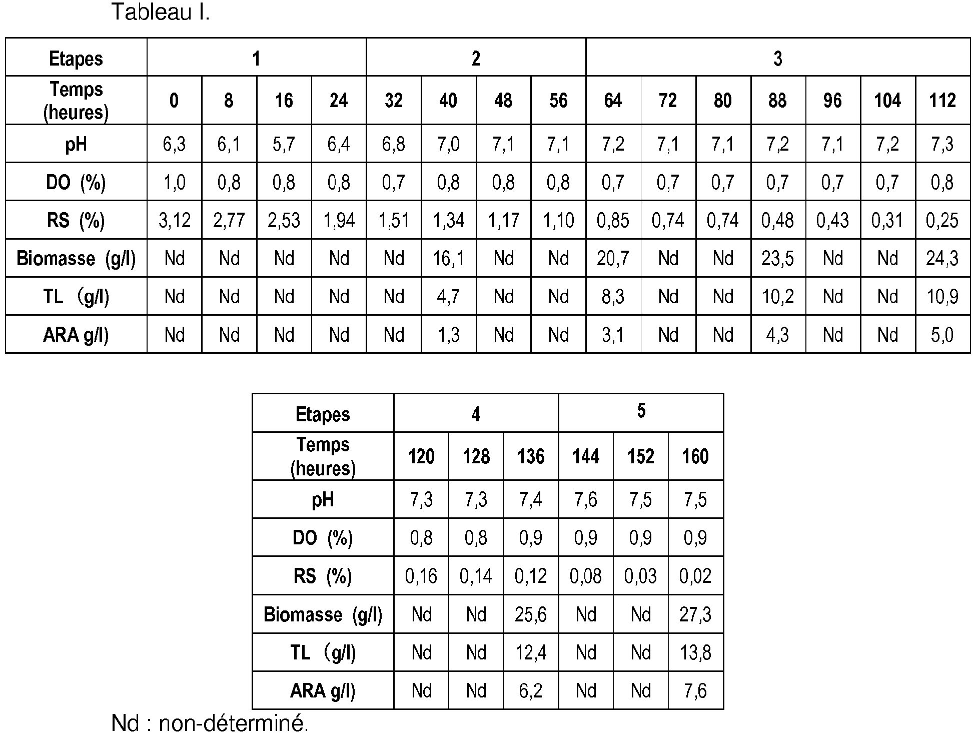

Le bilan de cette fermentation figure dans le tableau II suivant.

**Tableau II**

| Résultat moyen mesuré sur 10 "batches" de 85 m³ | |
|---|---|
| Durée de Fermentation | 165 heures |
| Volume final | 62 m³ |
| Contenu en lipides | 12.3 g/l |
| Contenu en ARA | 55 % |
| contenu en EPA | 0,1 % |
| Productivité en ARA brut | **0,04 g/l/h** |

### Exemple 2 : Récupération et conditionnement de la biomasse de la souche CNCM I-4642

À la fin de la cinquième étape de la conduite de fermentation de l'exemple 1, après que le milieu ait été pasteurisé 30 minutes à 70°C et après refroidissement à 30 - 25°C, les micro-organismes peuvent être ensuite récupérés du milieu de fermentation après déshydratation mécanique par filtration sous presse.

Après filtration et lavage avec de l'eau propre (0,5 V d'eau / 1 V de milieu), la teneur en matière sèche du gâteau de biomasse est de 65 à 75 %.

Dans une deuxième étape, le gâteau de biomasse est granulé en particules de 0,5 à 1,5 cm², puis les particules sont séchées en utilisant un séchoir à lit fluidisé.

Le temps de séchage est d'environ 45 à 55 minutes avec une température d'entrée d'air mis en place à 150°C.

Lorsque la température de l'air de sortie dépasse de 95 à 100°C, l'entrée d'air est arrêtée.

La biomasse est refroidie à la température ambiante et la biomasse est stockée en sac de 25 kg sous azote pour limiter l'oxydation.

La composition de la biomasse est présentée dans le tableau III suivant.

**Tableau III.**

| Résultat moyen sur 10 "batches" de 85 m³ | |
|---|---|
| Biomasse sèche (kg) | 1650 (rendement de récupération de 98%) |
| Humidité (%) | 6 |
| Lipides totaux (g/100g) | 44 (682 Kg/batch) |
| ARA (%) | 52 (355 kg/batch) |

### Exemple 3. Extraction de l'huile brute à partir de la biomasse séchée et granulée.

La technologie consiste à extraire l'huile par affinité avec un solvant liquide.

L'huile est extraite des granulés obtenus dans l'exemple 2 en utilisant du butane liquide sous pression de 6 à 7 bars.

Pour optimiser le rendement d'extraction, la biomasse est mélangée successivement sept fois avec du solvant frais recyclé, et chaque fois le temps de contact est de 50 à 60 min.

Le rendement d'extraction est de plus de 85 % (et peut atteindre des valeurs comprises entre 90 et 95 %).

L'huile est récupérée après distillation du solvant sous vide et séchage (Pression de 0,1 mPa et Température de 70 à 80°C).

Le Tableau IV suivant présente le profil de l'huile brute obtenue.

**Tableau IV.**

| Résultat moyen sur 10 "batches" de 85 m³ | |
|---|---|
| Rendement d'extraction (%) | 86 |
| Humidité (%) | 0,1 |
| Lipides totaux (g/100g) | 99,9 (585 Kg/batch) |
| Triglycérides (% poids) | 87 à 93 |
| Acides gras saturés (% poids) | 20 à 23 |
| ARA (%) | 52 (304 kg/batch) |
| Acidité (mg KOH/g) | 2 |
| Indice de peroxydes (meq/kg) | 5,5 |
| Teneur en insaponifiables (%) | 5,3 |
| Phosphore (ppm) | < 100 |
| EPA (%) | 0 |

### Exemple 4. Raffinage et purification de l'huile

### 1. Phase de raffinage

Cette phase de raffinage comprend six étapes successives classiquement mises en oeuvre par l'homme du métier:
- dégommage : acidification à l'acide citrique,
- saponification : neutralisation avec alcalis,
- centrifugation pour éliminer les gommes et les savons,
- lavage à l'eau,
- décoloration avec de la terre de silice, du charbon actif et de l'argile,
- filtration.

### 2. Phase de purification

Cette phase de purification a pour objectif d'éliminer les mauvais goûts, les peroxydes et optimise la stabilité de l'huile.

Cette phase comprend successivement une ou deux étapes de :
- distillation moléculaire (utilisée si le facteur UNS est trop élevé),
- désodorisation à la vapeur sous vide poussé.

Sur la totalité du procédé, le rendement de purification est de l'ordre de 78 à 80 %, et les indicateurs de performance sont présentés dans le tableau V suivant :

**Tableau V.**

| Résultat moyen sur 10 "batches" de 85 m³ | |
|---|---|
| Rendement de raffinage (%) | 79 |
| Humidité (%) | ≤ 0,01 |
| Lipides totaux (g/100g) | 99,99 (462 Kg/batch) |
| Triglycérides (wt%) | ≥ 90 |
| Acides gras saturés (wt%) | 20 à 23 |
| ARA (%) | ≥ 50 (231 kg/batch) |
| Acidité (mg KOH/g) | ≤ 1 |
| Indice de peroxydes (meq/kg) | ≤ 2 |
| Teneur en insaponifiables (%) | ≤ 3 |
| Humidité + matières volatiles (%) | ≤ 0,1 |
| Impuretés (%) | ≤ 0,1 |
| Solvant résiduel (ppm) | < 1 |
| PUFA (%) | ≥ 65 |
| EPA (%) | 0 |
| Trans FA (wt%) | < 1 |

| | |
|---|---|
| PUFA : PolyUnsaturated Fatty Acids (acides gras polyinsaturés) Trans FA : Trans Fatty Acids (acides gras trans) | |

L'huile finale se présente sous la forme d'un liquide jaune clair à 40°C, homogène et sans impuretés étrangères.

L'odeur est neutre, sans flaveur rance.

Est également récupéré la fraction volatile de l'étape de désodorisation à la vapeur sous vide poussé.

Classiquement, cette étape de désodorisation est réalisée dans un réacteur spécifique, où l'huile est chauffée et subit un passage sous vide poussé afin de libérer la fraction volatile.

Cette technologie batch est mise en oeuvre ici dans un réacteur de 1200 l avec un taux de charge de 50 %, sous atmosphère inerte à l'azote.

L'huile est chauffée progressivement de la température ambiante à 185°C (température amenée par la double enveloppe et par injection interne de vapeur à 190°C).

Après stabilisation de la température à 185°C, le vide est fait progressivement jusqu'à 260 Pa et est maintenu 30 minutes.

La fraction gazeuse libérée par ce traitement est condensée à température ambiante et collectée par un cyclone externe.

La composition de cette fraction est déterminée par :
- spectrométries Infrarouge et de RMN du proton à 25°C, en solution dans CDCL₃+CD₃OD et du phosphore à 25°C en solution dans CDCL₃+CD₃OD + tampon pH 7, et
- chromatographie en Phase Gazeuse selon la F-CPG-043 pour les acides gras totaux.

La fraction est constituée majoritairement de triglycérides (teneur estimée à environ 80%), du squalène étant détecté à hauteur de 10%.

Le profil des acides gras est le suivant :

| **NOMENCLATURE** | **NOMENCLATURE ABREGEE** | | g/100g brut |
|---|---|---|---|
| | Chimie | Physiologie | |
| laurique | C12:0 | | < 0,1 |
| myristique | C14:0 | | < 0,3 |
| pentadécylique | C15:0 | | < 0,1 |
| palmitique | C16:0 | | 5,6 |
| palmitoléique | C16:1 Δ9c | | < 0,3 |
| stéarique | C18:0 | | 8,5 |
| oléique | C18:1 Δ9c | n-9 (w9) | 7,0 |
| linoléique (LA), | C18:2 Δ9c, 12c | **n-6 (w6)** | 9,3 |
| γ-linolénique (GLA) | C18:3 Δ6c, 9c, 12c | **n-6 (w6)** | 2,1 |
| α-linolénique (ALA), | C18:3 Δ9c, 12c, 15c | **n-3 (w3)** | < 0,3 |
| arachidique | C20:0 | | 0,9 |
| stéaridonique (SDA, STD) | C18:4 Δ6c, 9c, 12c, 15c | **n-3 (w3)** | <0,1 |
| gondoique | C20:1 Δ11c | n-9 (w9) | <0,3 |
| dihomo-gamma-linolenic acid (DGLA) | C20:3 Δ8c, 11c, 14c | **n-6 (w6)** | 1,6 |
| **arachidonique (AA)** | C20:4 Δ5c, 8c, 11c, 14c | **n-6 (w6)** | 39,2 |
| (ETE) | C20:3 Δ11c, 14c, 17c | **n-3 (w3)** | <0,1 |
| béhénique | C22:0 | | 1,7 |
| **timnodonique (EPA)** | C20:5 Δ5c, 8c, 11c, 14c, 17c | **n-3 (w3)** | <0,3 |
| lignocérique | C24:0 | | 2,1 |
| (Osbond acid) | C22:5 Δ4c, 7c, 10c, 13c, 16c | **n-6 (w6)** | < 0,1 |
| nervonique | C24:1 Δ15c | n-9 (w9) | < 0,1 |
| clupanodonique (DPA) | C22:5 Δ7c, 10c, 13c, 16c, 19c | **n-3 (w3)** | < 0,1 |
| **cervonique (DHA)** | C22:6 Δ4c, 7c, 10c, 13c, 16c, 19c | **n-3 (w3)** | < 0,1 |
| | autres | | |
| | total | | 78,5 |

Limite de quantifiquation : 0,3%/brut
Limite de détection : 0,1%/brut

### Exemple 5. Etude comparative du profil lipidique de l'huile conforme à la description en regard de ceux des huiles du commerce ou décrites dans la littérature

Les acides gras ont été déterminés par chromatographie en phase gazeuse sous la forme d'esters méthyliques après transestérification au méthanol chlorhydrique et extraction au chloroforme. Les résultats sont exprimés en distribution, en % ; l'analyse est réalisée par la méthode de normalisation interne.

Un chromatographe (Type VARIAN 3800) équipé d'un injecteur split-splitless muni d'un tapfocus liner et d'un détecteur à ionisation de flamme a été utilisé.

Une solution d'étalon interne à environ précisément 0.5 mg de méthylheptadécanoate par ml de méthanol a été préparée. Le méthylheptadécanoate a servi de repère chromatographique.

Dans un tube de 6 ml, on a pesé environ précisément 30 mg d'échantillon séché au préalable. On a ajouté à la pipette à 2 traits 1 ml de la solution d'étalon interne puis 2 ml de méthanol chlorhydrique 3N. On a ensuite bouché et placé au bain à sec thermostaté à 110°C pendant 4 h.

Après refroidissement, on a ajouté environ 0.5 ml d'eau et 0.5 ml d'eau saturée en chlorure de sodium, extraire par 3 fois 1 ml de chloroforme. On a récupéré les phases chloroformiques dans un tube de 6 ml en les séchant sur une colonne contenant du sulfate de sodium. On a concentré sous courant d'azote jusqu'à 1 mL environ et injecté.

La distribution de chaque acide gras (i), en %, a été obtenu par le ratio de la surface du pic de cet acide gras par rapport à la somme des surfaces de tous les pics repérés sur le chromatogramme, de l'acide laurique (C12:0) au DHA (C22:6 Δ4c, 7c, 10c, 13c, 16c, 19c) inclus, en excluant le pic du méthylheptadécanoate.

Les huiles analysées selon cette méthode (dans le tableau VI suivant), outre celles de la description, sont des huiles commercialisées par les sociétés CARGILL, FUXING et SUNTORY.

En témoin sont présentés les profils en acides gras des huiles extraites de *M. carmagensis* et *M. schmuckeri* décrits dans la littérature.

Les huiles riches en arachidonique conformes à la description présente :
- plus de 50 % d'ARA
- une teneur en EPA de l'ordre de 0,1 %
- et surtout, par rapport aux autres huiles issues de microorganismes du commerce ou décrites dans la littérature, moins de 0,5 %, de préférence moins de 0,2 % d'acide myristique (C14:O), moins de 9 %, de préférence moins de 7 % d'acide palmitique (C16:0), moins de 3 %, de préférence moins de 2,5 % d'acide béhénique (C22:0) et moins de 3 %, de préférence moins de 2,5 % d'acide lignocérique (C 24:0).

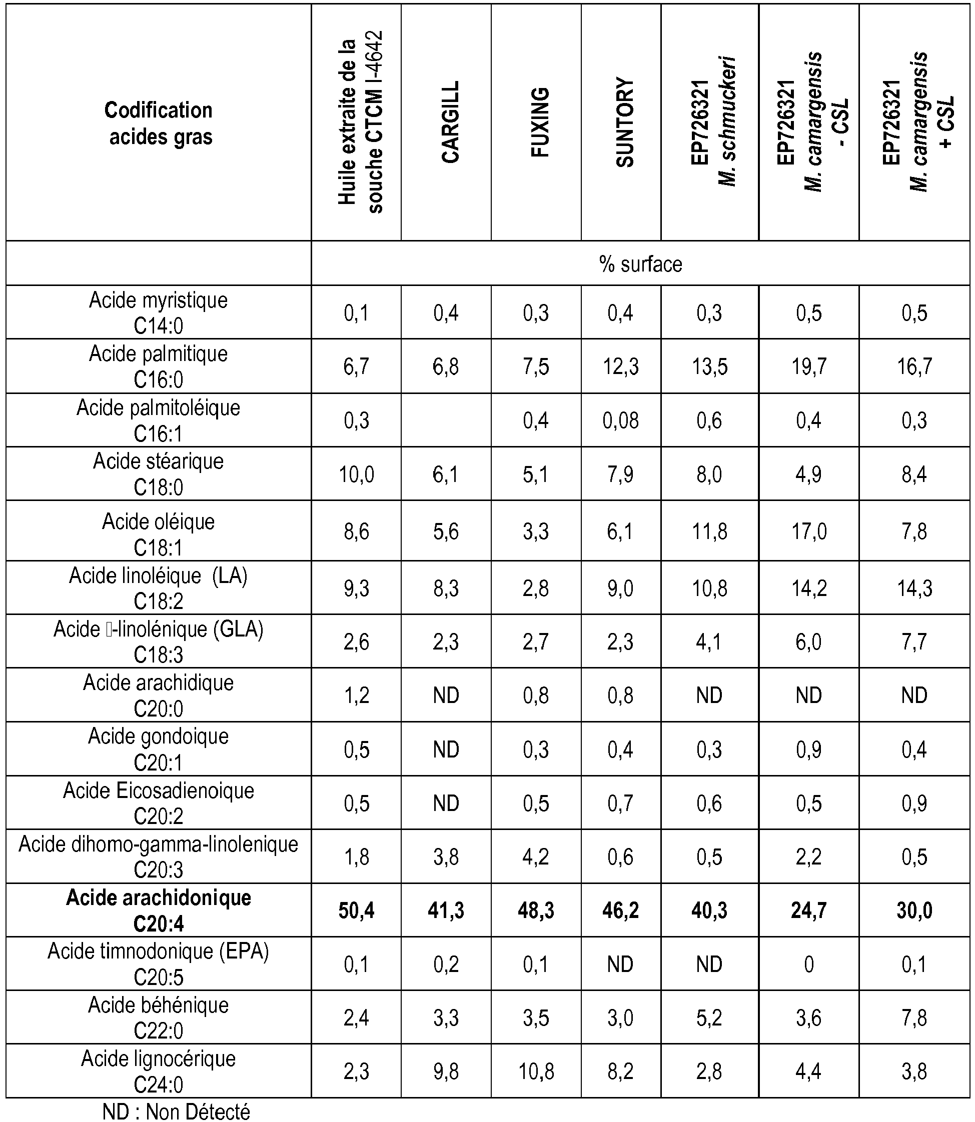

### Exemple 6. Caractérisation de la biomasse résiduelle obtenue après l'étape d'extraction de l'huile.

Après les étapes de raffinage et de purification de l'exemple 4, la biomasse résiduelle obtenue présente une matière sèche comprise entre 85 et 95 %.

Des analyses sont alors menées afin d'en déterminer la teneur en :
- azote total (détermination du % en N 6,25)
- lipides totaux,
- sucres totaux,
- fibres solubles
et également :
- la répartition en acides gras résiudels,
- le profil des sucres résiduels,
- l'aminogramme.

Le tableau VII suivant présente le profil de 5 lots de biomasse résiduelle.

| **Biomasse ARA** | Unités | **lot 1** | **lot 2** | **lot 3** | **lot 4** | **lot 5** |
|---|---|---|---|---|---|---|
| **Matière sèche** | % | **87,60** | **90,90** | **91,20** | **91,70** | **90,90** |
| **Nx6,25** | %/brut | **33,80** | **36,20** | **37,30** | **36,50** | **36,50** |
| **Résidu à la calcination** | %/brut | **4,50** | **4,70** | **4,80** | **4,60** | **4,70** |
| **Lipides Totaux** | %/brut | **12,10** | **12,10** | **11,50** | **11,80** | **12,70** |
| **Fibres (méthode PROSKY)** | %/brut | **23,20** | **23,00** | **25,00** | **26,40** | **24,10** |
| | | | | | | |

| **Profil en acides gras** | | | | | | |
|---|---|---|---|---|---|---|
| C12:0 laurique | %/brut | <0,03 | <0,03 | <0,03 | <0,03 | <0,03 |
| C14:0 myristique | %/brut | <0,1 | <0,1 | <0,1 | <0,1 | <0,1 |
| C15:0 pentadecylique | %/brut | <0,03 | <0,03 | <0,03 | <0,03 | <0,03 |
| C16:0 palmitique | %/brut | 1,40 | 1,40 | 1,20 | 1,40 | 1,40 |
| C16:1 Δ9c palmitoleique | %/brut | 0,20 | 0,20 | 0,20 | 0,20 | 0,20 |
| C18:0 stearique | %/brut | 1,00 | 1,10 | 1,00 | 1,10 | 1,10 |
| C18:1 Δ9c oleique | %/brut | 1,10 | 1,20 | 0,90 | 1,20 | 1,20 |
| C18:2 Δ9c, 12c linoleique (LA) | %/brut | 0,90 | 1,00 | 0,80 | 1,00 | 0,90 |
| C18:3 Δ9c, 12c, 15c a-linolenique (ALA) | %/brut | <0,03 | <0,03 | <0,03 | <0,03 | <0,03 |
| C18:3 Δ6c, 9c, 12c g-linolenique (GLA) | %/brut | 0,50 | 0,50 | 0,40 | 0,50 | 0,50 |
| C18:4 Δ6c, 9c, 12c, 15c stearidonique (SDA, STD) | %/brut | <0,03 | <0,03 | <0,03 | <0,03 | <0,03 |
| C20:0 arachidique | %/brut | <0,1 | <0,1 | <0,1 | <0,1 | <0,1 |
| C20:1 Δ11c gondoique | %/brut | <0,1 | <0,1 | <0,1 | <0,1 | <0,1 |
| C20:3 Δ8c, 11c, 14c dihomo-g-linolenique (DGLA) | %/brut | 0,30 | 0,30 | 0,20 | 0,30 | 0,30 |
| C20:3 Δ11c, 14c, 17c (ETE) | %/brut | <0,03 | <0,03 | <0,03 | <0,03 | <0,03 |
| C20:4 Δ5c, 8c, 11c, 14c arachidonique (AA) | %/brut | 4,40 | 4,00 | 4,50 | 3,80 | 4,20 |
| C20:5 Δ5c, 8c, 11c, 14c, 17c timnodonique (EPA) | %/brut | <0,03 | <0,03 | <0,03 | <0,03 | <0,03 |
| C22:0 behenique | %/brut | 0,20 | 0,20 | 0,20 | 0,20 | 0,20 |
| C22:5 Δ7c, 10c, 13c, 16c, 19c clupanodonique (DPA) | %/brut | <0,03 | <0,03 | <0,03 | <0,03 | <0,03 |
| C22:5 Δ4c, 7c, 10c, 13c, 16c (acide Osbond) | %/brut | <0,03 | <0,03 | <0,03 | <0,03 | <0,03 |
| C22:6 Δ4c, 7c, 10c, 13c, 16c, 19c cervonique (DHA) | %/brut | <0,03 | <0,03 | <0,03 | <0,03 | <0,03 |
| C24:0 lignocerique | %/brut | 0,40 | 0,40 | 0,40 | 0,40 | 0,40 |
| C24:1 Δ15c nervonique | %/brut | <0,03 | <0,03 | <0,03 | <0,03 | <0,03 |
| Autres acides gras | %/brut | 0,10 | 0,10 | 0,10 | 0,10 | 0,10 |
| **∑ AGT** | **%/brut** | **10,60** | **10,50** | **10,00** | **10,30** | **10,60** |
| | | | | | | |

| **Profil en sucres** | | | | | | |
|---|---|---|---|---|---|---|
| Arabinose | %/brut | <0,1 | <0,1 | <0,1 | <0,1 | <0,1 |
| Galactose | %/brut | 1,20 | 1,00 | 1,00 | 1,10 | 1,10 |
| Glucose | %/brut | 13,50 | 15,90 | 15,00 | 16,10 | 15,10 |
| Mannose | %/brut | 1,70 | 1,80 | 1,90 | 1,80 | 1,90 |
| Rhamnose | %/brut | nd | nd | nd | nd | nd |
| Ribose | %/brut | 0,30 | 0,30 | 0,30 | 0,30 | 0,30 |
| Xylose | %/brut | 0,10 | 0,10 | 0,10 | 0,10 | 0,10 |
| **∑ Sucres Totaux** | **%/brut** | **16,80** | **19,10** | **18,30** | **19,40** | **18,50** |
| | | | | | | |

| **Aminogramme** | | | | | | |
|---|---|---|---|---|---|---|
| Acide Aspartique | %/brut | 2,06 | 2,34 | 2,41 | 2,42 | 2,35 |
| Acide Glutamique | %/brut | 2,82 | 3,15 | 3,13 | 3,19 | 3,06 |
| Alanine | %/brut | 1,40 | 1,56 | 1,44 | 1,58 | 1,48 |
| Arginine | %/brut | 1,68 | 2,00 | 2,39 | 1,98 | 2,21 |
| Cystine | %/brut | 0,39 | 0,43 | 0,48 | 0,44 | 0,45 |
| Glycine | %/brut | 1,07 | 1,23 | 1,16 | 1,21 | 1,17 |
| Histidine | %/brut | 1,08 | 1,24 | 1,21 | 1,23 | 1,13 |
| Isoleucine | %/brut | 0,94 | 1,06 | 0,99 | 1,05 | 1,01 |
| Leucine | %/brut | 2,40 | 2,78 | 3,12 | 2,73 | 2,71 |
| Lysine | %/brut | 2,05 | 2,38 | 2,39 | 2,34 | 2,31 |
| Methionine | %/brut | 0,49 | 0,51 | 0,44 | 0,47 | 0,45 |
| Phenylalanine | %/brut | 1,64 | 1,90 | 2,03 | 1,87 | 1,81 |
| Proline | %/brut | 0,89 | 1,02 | 1,07 | 1,10 | 1,08 |
| Serine | %/brut | 1,10 | 1,27 | 1,28 | 1,27 | 1,25 |
| Threonine | %/brut | 1,17 | 1,29 | 1,31 | 1,32 | 1,30 |
| Tryptophane | %/brut | 0,65 | 0,69 | 0,93 | 0,71 | 0,74 |
| Tyrosine | %/brut | 0,87 | 0,96 | 1,05 | 1,00 | 1,01 |
| Valine | %/brut | 1,33 | 1,47 | 1,53 | 1,48 | 1,46 |
| **∑ AAT** | **%/brut** | **24,00** | **27,30** | **28,40** | **27,40** | **27,00** |
| | | | | | | |

| **Teneur en métaux lourds** | | | | | | |
|---|---|---|---|---|---|---|
| Plomb | mg/kg | 0,04 | 0,04 | 0,05 | 0,04 | 0,03 |
| Cadmium | mg/kg | 0,06 | 0,07 | 0,07 | 0,06 | 0,07 |
| Mercure | mg/kg | < 0,01 | < 0,01 | < 0,01 | < 0,01 | < 0,01 |
| Arsenic | mg/kg | 0,12 | 0,11 | 0,11 | 0,11 | 0,11 |
| Zinc | mg/kg | 22 | 23 | 23 | 22 | 25 |
| Chrome | mg/kg | 8,9 | 2 | 1,4 | 1,2 | 2 |
| Manganese | mg/kg | 6,8 | 6,9 | 6,9 | 6,6 | 7 |

La biomasse résiduelle apparait donc tout à fait adaptée à son application en nutrition animale, tant par sa teneur en azote protéique (entre 35 et 45 %), sa teneur en fibres solubles (entre 20 et 30 %), en sucres résiduels (entre 15 et 20%) et pour certaines espèces spécifiques (animaux de compagnie, aquaculture), pour sa teneur résiduelle en ARA (entre 3,5 et 4,5 %).

## Revendications

1. Souche de *Mortierella* alpina déposée le 12 juin 2012 auprès de la CNCM sous le numéro I-4642.

2. Méthode de production des composés lipidiques d'intérêt comprenant une culture de la souche selon la revendication 1 et la récupération de la biomasse riche en composés lipidiques d'intérêt et, facultativement, la récolte des composés lipidiques d'intérêt.

3. Méthode selon la revendication 2, dans laquelle le composé lipidique d'intérêt est l'acide arachidonique (ou ARA).

4. Méthode selon la revendication 3, dans laquelle l'acide arachidonique (ARA) se présente sous la forme d'une huile.

5. Méthode selon la revendication 4, **caractérisée en ce que** l'huile contenant l'acide arachidonique est préparée par une méthode comprenant :
∘ culture de la souche en conditions hétérotrophiques de manière à produire une biomasse présentant entre 40 et 55 % en poids de lipides, préférentiellement entre 40 et 50 % en poids de lipides, plus préférentiellement encore de l'ordre de 45 % en poids de lipides et entre 50 et 55 % en poids d'ARA sur acides gras totaux.
∘ récolte de la biomasse ainsi préparée
∘ séchage de ladite biomasse
∘ extraction de l'huile par solvant choisi dans le groupe constitué de l'hexane et du butane, plus particulièrement par du butane liquide, et
∘ raffinage et récupération de l'huile ainsi extraite.

6. Utilisation de la souche selon la revendication 1 pour produire des composés lipidiques d'intérêt, tel l'acide arachidonique (ou ARA).

7. Produit ou composition comprenant la souche selon la revendication 1, ou un lysat de celle-ci riche en composés lipidiques d'intérêt, **caractérisé en ce qu'**il est riche en acide arachidonique (ou ARA) et qu'il comprend plus de 50 % d'acide arachidonique (ou ARA) en poids sur acide gras totaux, et présente :
- moins de 0,5 %, de préférence moins de 0,2 % d'EPA,
- moins de 0,5 %, de préférence moins de 0,2 % d'acide myristique (C14:O),
- moins de 9 %, de préférence moins de 7 % d'acide palmitique (C16:0),
- moins de 3 %, de préférence moins de 2,5 % d'acide béhénique (C22:0) et
- moins de 3 %, de préférence moins de 2,5 % d'acide lignocérique (C 24:0)..

8. Produit ou composition selon la revendication 7, **caractérisé en ce qu'**il s'agit d'un produit alimentaire ou complémentaire alimentaire.

9. Méthode de préparation d'une souche de *Mortierella alpina* capable de produire des teneurs en acide arachidonique de plus de 50 % en poids sur acides gras totaux comprenant la mutagenèse ou la transformation génique de la souche selon la revendication 1.

## Patentansprüche

1. *Mortierella alpina* Stamm, hinterlegt am 12. Juni 2012 bei der CNCM unter der Nummer I-4642.

2. Verfahren zur Herstellung von Lipidverbindungen von Interesse, umfassend das Kultivieren des Stammes nach Anspruch 1 und das Gewinnen der an Lipidverbindungen von Interesse reichen Biomasse und gegebenenfalls das Ernten der Lipidverbindungen von Interesse.

3. Verfahren nach Anspruch 2, wobei die Lipidverbindung von Interesse Arachidonsäure (oder ARA) ist.

4. Verfahren nach Anspruch 3, wobei die Arachidonsäure (ARA) in Form eines Öls vorliegt.

5. Verfahren nach Anspruch 4, **dadurch gekennzeichnet, dass** das Arachidonsäure enthaltende Öl durch ein Verfahren hergestellt wird, umfassend:
∘ Kultivierung des Stammes unter heterotrophen Bedingungen, um eine Biomasse mit 40 bis 55 Gewichts-% Lipiden herzustellen, bevorzugt zwischen 40 und 50 Gewichts-% Lipiden, noch weiter bevorzugt etwa 45 Gewichts-% Lipiden und zwischen 50 und 55 Gew.-% ARA bezogen auf die Gesamtmenge an Fettsäuren,
∘ Ernten der so hergestellten Biomasse,
∘ Trocknen der Biomasse,
∘ Extrahieren des Öls durch Lösungsmittel ausgewählt aus der Gruppe bestehend aus Hexan und Butan, insbesondere durch flüssiges Butan, und
∘ Raffination und Gewinnung des so extrahierten Öls.

6. Verwendung des Stammes nach Anspruch 1 zur Herstellung von Lipidverbindungen von Interesse, wie etwa Arachidonsäure (oder ARA).

7. Produkt oder Zusammensetzung, umfassend den Stamm nach Anspruch 1 oder ein Lysat davon, welches reich an Lipidverbindungen von Interesse, wie Arachidonsäure (oder ARA) ist, **dadurch gekennzeichnet, dass** es mehr als 50% Arachidonsäure (oder ARA) enthält, bezogen auf die Gesamtmenge an Fettsäuren und mit:
- weniger als 0,5%, bevorzugt weniger als 0,2% EPA,
- weniger als 0,5%, bevorzugt weniger als 0,2% Myristinsäure (C14:0),
- weniger als 9%, bevorzugt weniger als 7% Palmitinsäure (C16:0),
- weniger als 3%, bevorzugt weniger als 2,5% Behensäure (C22:0) und
- weniger als 3%, bevorzugt weniger als 2,5% Lignocerinsäure (C24:0).

8. Produkt oder Zusammensetzung nach Anspruch 7, **dadurch gekennzeichnet, dass** es ein Nahrungsmittel oder Nahrungsergänzungsmittel ist.

9. Verfahren zur Herstellung eines Stammes von *Mortierella alpina* der in der Lage ist, Arachidonsäuregehalte von mehr als 50 Gewichts-% herzustellen, bezogen auf die Gesamtmenge an Fettsäuren, umfassend Mutagenese oder Gentransformation des Stamms gemäß Anspruch 1.

## Claims

1. Strain of *Mortierella* alpina filed on 12 June 2012 with the CNCM under the number 1-4642.

2. Method for producing the lipid compounds of interest comprising a culture of the strain according to claim 1 and recovering the biomass rich in lipid compounds of interest and, optionally, harvesting the lipid compounds of interest.

3. Method according to claim 2, wherein the lipid compound of interest is arachidonic acid (or ARA).

4. Method according to claim 3, wherein the arachidonic acid (ARA) is in the form of an oil.

5. Method according to claim 4, **characterised in that** the oil containing the arachidonic acid is prepared by a method comprising:
- culture of the strain under heterotrophic conditions so as to produce a biomass having between 40 and 55% lipids by weight, preferably between 40 and 50% lipids by weight, even more preferably approximately 45% lipids by weight and between 50 and 55% ARA by weight with respect to total fatty acids.
- harvesting the biomass thus prepared
- drying of said biomass
- extraction of the oil by a solvent chosen from the group consisting of hexane and butane, more particularly by liquid butane, and
- refining and recovery of the oil thus extracted.

6. Use of the strain according to claim 1 to produce lipid compounds of interest, such as arachidonic acid (or ARA).

7. Product or composition comprising the strain according to claim 1, or a lysate of the latter rich in lipid compounds of interest, **characterised in that** it is rich in arachidonic acid (or ARA) and that it comprises more than 50% arachidonic acid (or ARA) by weight with respect to total fatty acids, and has:
- less than 0.5%, preferably less than 0.2% EPA,
- less than 0.5%, preferably less than 0.2% myristic acid (C14:O),
- less than 9%, preferably less than 7% palmitic acid (C16:0),
- less than 3%, preferably less than 2.5% behenic acid (C22:0) and
- less than 3%, preferably less than 2.5% lignoceric acid (C 24:0).

8. Product or composition according to claim 7, **characterised in that** it is a food product or a food supplement.

9. Method for preparing a strain of *Mortierella alpina* capable of producing arachidonic acid contents over 50% by weight with respect to total fatty acids and comprising mutagenesis or gene transformation of the strain according to claim 1.


### SEQUENCE LISTING

<110> ROQUETTE FRERES
<120> HUILE ENRICHIE EN ACIDE ARACHIDONIQUE ISSUE DE MICROORGANISMES (CHAMPIGNON UNICELLULAIRE Mortierella alpina) ET SON PROCEDE DE PREPARATION
<130> B1392PC
<160> 1
<170> PatentIn version 3.3
<210> 1
   <211> 361
   <212> DNA
   <213> Mortierella alpina
<400> 1
   ttaaacagtg cgtgaaattg ttgaaaggga aacgcttgac accagtcatg cgagcggaaa     60
   atcagtcttt tgcagtgggg agttgtgtgg gttcggaccg caaggccggc ctgtgctgca    120
   tctctgctgt aagtgatgca ctttttcgtt tgcaggccaa catcagtttc ttctgctgga    180
   caaaactctt gagaaggtag cagctttggc tgtgttatag ctcttgagcg atacagtgga    240
   ggggactgag gttttcgcag cgcgtgctct cgggcaaggc tgattgggtg ctatgggatc    300
   gttcggtgta caatgcatgc attttgcgcc gtgtcttttc tgtactcgct caactcggct    360
   c                                                                    361